# EUROPÄISCHE PATENTSCHRIFT

(11) **EP 0 729 761 B1**
(45) Veröffentlichungstag und Bekanntmachung des Hinweises auf die Patenterteilung: **01.08.2001**
(21) Anmeldenummer: 96100698.8
(22) Anmeldetag: 18.01.1996
(51) Int. Cl.: A61L 29/00, A61L 31/00, A61M 39/08, B32B 27/40

(54) **Schlauch für einen medizinischen Behälter**
Tube for medical container
Tuyau pour conteneur médical

(30) Priorität: 10.02.1995 DE 19504414
(43) Veröffentlichungstag der Anmeldung: 04.09.1996
(73) Patentinhaber: Fresenius Kabi AB, 751 75 Uppsala (SE)
(72) Erfinder: Wolkenstörfer, Reinhold, D-91077 Neunkirchen am Brand (DE); Iwatschenko, Peter, D-91077 Neunkirchen am Brand (DE)
(74) Vertreter: Luderschmidt, Schüler & Partner GbR

(56) Entgegenhaltungen:
- EP-A- 0 355 711
- EP-A- 0 380 270
- EP-A- 0 603 675
- WO-A-92/18173

## Beschreibung

Die Erfindung betrifft einen Schlauch für einen medizinischen Behälter sowie einen medizinischen Behälter mit einem Schlauchansatz. Als "medizinische Behälter" werden in diesem Zusammenhang alle Behälter, insbesondere Flaschen und Beutel bezeichnet, die für medizinische Zwecke eingesetzt werden. Dazu gehören insbesondere Behälter für die enterale oder parenterale Ernährung, für die Infusion von Medikamenten oder die Transfusion von Blut.

Medizinische Behälter sind insbesondere bekannt als Beutel, die bereits vom Hersteller mit einer lange lagerfähigen medizinischen Flüssigkeit gefüllt sind, und als sogenannte Mischbeutel. Letztere werden erst kurz vor der Anwendung mit einer Mischung aus mehreren medizinischen Flüssigkeiten gefüllt, und zwar entweder beim Hersteller/Lieferanten oder beim behandelnden Arzt. Mischbeutel werden insbesondere für solche medizinische Flüssigkeiten eingesetzt, die in gemischtem Zustand nicht lange haltbar sind.

Sowohl vorgefüllte Beutel als auch Mischbeutel weisen mindestens einen Ansatzstutzen auf, der aus einem kurzen Stück (einige Zentimeter) eines Schlauches gebildet ist. Dieser schlauchförmige Ansatz ist an einem Ende mit dem Beutel verbunden und am anderen Ende normalerweise mit einem Konnektor oder einem sonstigen Bauteil, insbesondere einem Hartbauteil, versehen. Der Konnektor oder das sonstige Bauteil können aus einem Kunststoff wie ABS, EVA oder PVC, aus Metall, einem anderen geeigneten Material oder einer Kombination dieser Materialien gebildet sein. Der Konnektor kann einen durchstechbaren Bereich aufweisen, beispielsweise eine Gummimembran oder einen Gummipfropfen. Zum Anschluß eines mit mindestens einem derartigen Konnektor versehenen Behälters wird eine Kanüle durch den durchstechbaren Bereich des Konnektors gestochen. Eine zusätzliche mechanische Verriegelung kann vorgesehen sein; sie ist aber nicht notwendig, weil die Kanüle von dem Gummimaterial des durchstechbaren Bereiches relativ fest gehalten wird. Die Kanüle kann mit einem längeren Schlauch verbunden sein, über den die medizinische Flüssigkeit einem Patienten zugeführt wird. An diesem letztgenannten Schlauch kann eine Regulierklemme zur Veränderung des Strömungsquerschnitts vorgesehen sein.

Ein Mischbeutel weist typischerweise drei der gerade beschriebenen Schlauchansätze auf, die jeweils mit einem Konnektor versehen sind. Die Ansätze dienen zum Befüllen des Beutels (hier ist ein spezieller, fest verschließbarer Metall- oder Kunststoffkonnektor vorgesehen), zum Entnehmen der medizinischen Flüssigkeit und zum Zugeben von weiteren Medikamenten zur im Beutel enthaltenen Flüssigkeit.

Ein erfindungsgemäßer Schlauch wird vorzugsweise bei der Herstellung eines medizinischen Behälters eingesetzt, um die oben beschriebenen kurzen Schlauchansätze des Behälters zu bilden. Jedoch ist ein erfindungsgemäßer Schlauch auch als Verbindungsleitung zwischen dem Behälter und dem Patienten verwendbar. Schließlich werden als Beutel ausgestaltete Behälter in der Regel aus langen Schläuchen mit verhältnismäßig großem Durchmesser durch Abtrennen und Verschweißen einzelner Stücke hergestellt. Auch solche Schläuche sollen als "Schläuche" im Sinne der Erfindung betrachtet werden.

Aus der EP 0 355 711 A1 ist ein aus einem homogenen Ethylen-Vinylacetat-Copolymer (EVA) bestehender Schlauch mit einem Vinylacetat-Anteil von 12 % bis 28 % bekannt, bei dem vorzugsweise das Ethylen-Vinylacetat-Copolymer nicht kreuzvernetzt ist.

Ein derartiger Schlauch weist gute mechanische Eigenschaften wie hohe Stabilität und Steifheit auf. Er ist außerdem chemisch inert und benötigt, beispielsweise im Gegensatz zu PVC, keine Weichmacher. Dies ist insbesondere bei der Verwendung mit fetthaltigen medizinischen Flüssigkeiten (beispielsweise bei Fettemulsionen zur künstlichen Ernährung) wichtig, da Weichmacher in Kunststoffen, die mit einer fetthaltigen medizinischen Flüssigkeit in Kontakt stehen, allmählich in dieser Flüssigkeit in Lösung gehen und sie verunreinigen.

Aus diesen Gründen ist es vorteilhaft, sowohl den Behälter als auch den oder die Anschlußstutzen aus EVA herzustellen. Diese Bauteile können miteinander verschweißt werden, beispielsweise durch Hochfrequenzschweißen.

Bei bekannten medizinischen Behältern mit Anschlußstutzen aus EVA ist in jeden der Anschlußstutzen ein zylindrisch-rohrförmiger Fortsatz des jeweiligen Konnektors oder des anderen Bauteils gesteckt. Es ist nicht möglich, das Bauteil mit dem Anschlußstutzen durch ein oberflächliches Anlösen der einander berührenden Flächen zu verkleben, weil das EVA-Material des Stutzens wegen seiner Inertheit nicht chemisch anlösbar ist.

Bei Behältern mit Anschlußstutzen aus EVA-Schlauch, in die die Konnektoren lediglich eingesteckt sind, tritt das Problem auf, daß die medizinische Flüssigkeit zwischen den einander zugewandten Oberflächen von Schlauch und Konnektor hindurchwandern und sich allmählich in der Wandung einlagern kann. Diese Erscheinung ist insbesondere bei fetthaltigen medizinischen Flüssigkeiten zu beobachten.

Aus der DE 33 18 730 A1 ist ein Verfahren zur Herstellung biokompatibler Polyurethane bekannt, die eine hohe Beständigkeit im menschlichen oder tierischen Körper aufweisen sollen.

Die DE 29 32 982 A1 offenbart eine thermoplastische Masse, die aus einer Mischung zweier unterschiedlicher EVA-Copolymere besteht. Ein aus dieser Masse gefertigter Schlauch zur Verabreichung eines medizinischen Fluidums an einen Patienten ist ebenfalls offenbart.

Aus der DE 30 26 974 A1 ist eine Kunststoffmasse bekannt, die sich gleichzeitig mit Polyvinylchloridkunststoffen und im wesentlichen aus Polyolefinen bestehenden Kunststoffen verschweißen läßt und die aus einer Mischung zweier Kunststoffe besteht. Auch die Verwendung einer derartigen Kunststoffmasse zur Herstellung eines Verbindungsteils bei einem biegsamen, zusammenlegbaren Behälter ist offenbart.

Demgemäß hat die Erfindung die Aufgabe, einen Schlauch für einen medizinischen Behälter bereitzustellen, der gute mechanische, gute chemische und gute verbindungstechnische Eigenschaften aufweist, was insbesondere heißt, daß eine zuverlässige und flüssigkeitsdichte Verbindung des Schlauches mit anderen Bauteilen möglich sein soll. Auch soll ein medizinischer Behälter mit einem derartigen Schlauch bereitgestellt werden.

Zur Lösung dieser Aufgaben sieht die Erfindung vor, einen Schlauch für einen medizinischen Behälter so auszugestalten, daß der Schlauch eine äußere Schicht aus Ethylen-Vinylacetat-Copolymer (EVA) und eine innere Schicht aus Polyurethan (PU) aufweist. Weiterhin sieht die Erfindung vor, einen medizinischen Behälter, insbesondere für die enterale oder parenterale Ernährung, mit mindestens einem Ansatz aus einem erfindungsgemäßen Schlauch zu versehen.

Ein erfindungsgemäßer Schlauch ist durch die äußere Schicht aus EVA mechanisch stabil und mit einem ebenfalls aus EVA bestehenden Behälter verschweißbar. Die innere Schicht aus PU ist fettkompatibel und damit auch für fetthaltige medizinische Flüssigkeiten geeignet. Im Gegensatz zu EVA läßt sich PU chemisch anlösen und deshalb mit anderen Bauteilen, beispielsweise Konnektoren aus ABS, PVC oder PU, durch eine Lösungsmittelklebung verbinden.

Die äußere Schicht des erfindungsgemäßen Schlauches kann einen Vinylacetat-Anteil von 10 % bis 30 % ausweisen, vorzugsweise von 14 % bis 28 %. Die %-Angaben beziehen sich jeweils auf Gewichtsprozent. Auch können der äußeren Schicht Zusätze von Thermoplasten und/oder Elastomeren hinzugefügt sein, zum Beispiel von Polyurethan, ABS oder thermoplastischem Polyester.

Zwar kann der erfindungsgemäße Schlauch aus zwei ineinandergeschobenen und flächig miteinander verbundenen Einzelschläuchen hergestellt sein; vorzugsweise ist jedoch der Schlauch durch Coextrusion hergestellt, also durch ein Verfahren, bei dem die beiden Schichten in einem Arbeitsgang von einer Coextrusionsmaschine in ihrer erfindungsgemäßen Anordnung stranggepreßt werden. Dadurch wird eine besonders feste und dichte Verbindung der Schichten erreicht.

Die äußere bzw. innere Schicht des Schlauches brauchen nicht dessen "äußerste" bzw. "innerste" Schicht zu sein. Vielmehr kann auf einer Innenfläche der PU-Schicht nochmals eine Abdeckschicht aufgetragen sein, die die sowieso schon guten chemischen Eigenschaften der PU-Schicht weiter verbessert. Ebenso kann auf einer Außenfläche der EVA-Schicht eine weitere Schicht angeordnet sein, beispielsweise ein durchscheinender farbiger Lack. Auch zwischen der äußeren und der inneren Schicht können sich weitere Schichten befinden, so etwa eine Klebstoffschicht. Vorzugsweise besteht der Schlauch aber aus genau zwei Schichten, so daß die äußere und innere Schicht unmittelbar aneinander anliegen und keine weiteren Materialien innerhalb der inneren Schicht oder außerhalb der äußeren Schicht vorgesehen sind. Dies ist die am wenigsten aufwendige Ausführungsform der Erfindung.

Vorzugsweise ist der Schlauch durch Behandlung mit Ethylenoxid oder durch Strahlensterilisation sterilisiert. Der erfindungsgemäße Schlauch weist ein gutes Desorptionsverhalten für Ethylenoxid auf, das heißt, nach der Sterilisation dauert es nur relativ kurz, bis das Ethylenoxid bis auf den zugelassenen Rest von 1 ppm desorbiert ist.

In einer bevorzugten Ausführungsform ist das Verhältnis der Wandstärke der inneren Schicht zur Wandstärke des Schlauches höchstens 0,5 und liegt vorzugsweise zwischen 0,25 und 0,4. Die äußere Schicht ist bei einem aus zwei Schichten bestehenden Schlauch dann dicker als die innere Schicht.

Die Wandstärke der inneren Schicht kann zwischen 0,2 mm und 0,5 mm betragen, vorzugsweise 0,35 mm. Die Wandstärke des Schlauches kann zwischen 1 mm und 3 mm betragen, vorzugsweise zwischen 1,6 mm und 2 mm, und das Verhältnis des Innendurchmessers zum Außendurchmesser des Schlauches kann zwischen 0,6 und 0,9 betragen, vorzugsweise 0,75. Ein Schlauch mit diesen Abmessungen ist hinreichend stabil.

In einer bevorzugten Ausführungsform beträgt der Außendurchmesser des Schlauches zwischen 2 mm und 10 mm, vorzugsweise zwischen 6 mm und 8 mm. Diese Dimensionierung ist für die Verwendung bei Infusionsbeuteln besonders geeignet.

Ein mit einem erfindungsgemäßen Schlauchstutzen und damit verklebten Konnektoren ausgestatteter medizinischer Behälter ist zuverlässig flüssigkeitsdicht und kostengünstig in der Herstellung. Dies gilt insbesondere, wenn der Behälter aus Ethylen-Vinylacetat-Copolymer besteht und mit der äußeren Schicht des Schlauches verschweißt ist, und/oder, wenn die innere Schicht des Schlauches mit einem Konnektor oder einem sonstigen Bauteil verklebt ist. Die letztgenannte Verklebung kann durch Anlösen des Schlauches und/oder des anderen Bauteils durch ein Lösungsmittel erfolgen.

Zwei Ausführungsbeispiele der Erfindung werden nun anhand der Zeichnung genauer beschrieben. Es stellen dar:
- Fig. 1: einen Querschnitt durch eine erste Ausführungsform eines erfindungsgemäßen Schlauches, und
- Fig. 2: einen Querschnitt durch eine zweite Ausführungsform eines erfindungsgemäßen Schlauches.

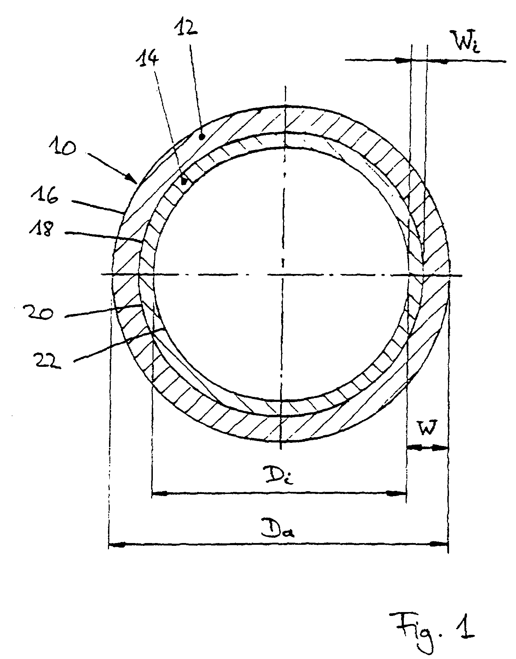

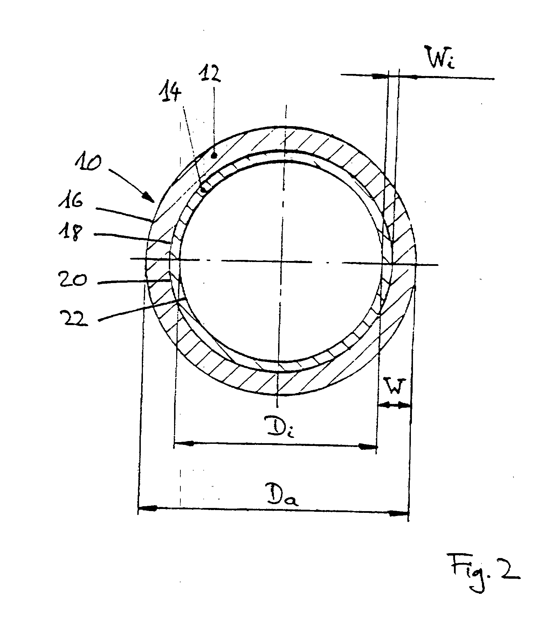

In Fig. 1 und Fig. 2 ist jeweils ein Schlauch 10 mit einer äußeren Schicht 12 und einer inneren Schicht 14 dargestellt. Die Schichten 12 und 14 haben eine gleichmäßige Wandstärke. Eine Außenfläche 20 der inneren Schicht 14 steht vollflächig in Kontakt mit einer Innenfläche 18 der äußeren Schicht 12. Die Außenfläche 20 und eine Innenfläche 22 der inneren Schicht 14 sowie eine Außenfläche 16 und die Innenfläche 18 der äußeren Schicht 12 haben jeweils einen kreisförmigen Querschnitt. Sie sind koaxial angeordnet, also mit im Querschnitt zusammenfallenden Mittelpunkten.

Die äußere Schicht 12 besteht aus einem Ethylen-Vinylacetat-Copolymer mit einem Vinylacetat-Anteil von 14 % bis 28 %. Die innere Schicht 14 besteht aus Polyurethan in einer medizinischen Qualität.

Der Schlauch ist durch Coextrusion hergestellt, so daß die äußere Schicht 12 und die innere Schicht 14 fest miteinander verbunden sind. Der Schlauch 10 ist auf der Innenfläche 22 der inneren Schicht 14 und der Außenfläche 16 der äußeren Schicht 12 durch Behandlung mit Ethylenoxid sterilisiert.

Bei der in Fig. 1 gezeigten Ausführungsform des Schlauches 10 beträgt der Innendurchmesser Dᵢ 6 mm (mit einer zulässigen Toleranz von ±0,15 mm), der Außendurchmesser Dₐ 8 mm (mit einer zulässigen Toleranz von +0,3 mm und -0,1 mm) und die Wandstärke Wᵢ der inneren Schicht 14 0,35 mm (mit einer zulässigen Toleranz von +0,1 mm). Dementsprechend ergibt sich bei der Ausführungsform nach Fig. 1 die Wandstärke W des Schlauches 10 zu ungefähr 1 mm. In dieser Ausführungsform hat der Schlauch 10 eine zulässige Koaxialität, also eine Abweichung der Mittelpunkte der kreisförmigen Querschnitte, von höchstens 0,11 mm.

Bei der in Fig. 2 gezeigten Ausführungsform des Schlauches 10 beträgt der Innendurchmesser Dᵢ 4,8 mm (mit einer zulässigen Toleranz von ±0,1 mm), der Außendurchmesser Dₐ 6,4 mm (mit einer zulässigen Toleranz von +0,3 mm und -0,1 mm) und die Wandstärke Wᵢ der inneren Schicht 14 0,35 mm (mit einer zulässigen Toleranz von +0,1 mm). Dementsprechend ergibt sich bei der Ausführungsform nach Fig. 2 die Wandstärke W des Schlauches 10 zu ungefähr 0,8 mm. In dieser Ausführungsform hat der Schlauch 10 eine zulässige Koaxialität von höchstens 0,09 mm.

Ein in den Figuren nicht dargestelltes Infusionsgerät weist einen medizinischen Behälter und einen Verbindungsschlauch zum Patienten auf. Der medizinische Behälter weist einen Beutel, einen Anschlußstutzen und einen Konnektor auf. Der Beutel besteht aus Ethylen-Vinylacetat-Copolymer und ist mit einer fetthaltigen Ernährungslösung gefüllt. Der aus einem einige Zentimeter langen Stück des Schlauches 10 gebildete Anschlußstutzen ist an einem Ende an der äußeren Schicht 12 mit dem Beutel verschweißt. Der Konnektor besteht aus einem ABS-Formstück oder einem Formstück aus einem anderen Kunststoff mit einer durchgehenden Bohrung, die auf einer Seite den Hohlraum eines rohrartigen Fortsatzes des Konnektors bildet und auf der anderen Seite von einer durchstechbaren Gummiplatte verschlossen ist. Der rohrförmige Fortsatz ist in das andere Ende des Schlauches 10 geschoben und dort durch eine Lösungsmittelklebung mit der inneren Schicht 14 des Schlauches 10 verbunden.

Zur Herstellung des Schlauches 10 werden einer Coextrusionsmaschine die beiden Grundstoffe Ethylen-Vinylacetat-Copolymer und Polyurethan als Feststoffe, insbesondere als Granulate, getrennt zugeführt. Die beiden Stoffe werden erwärmt und von je einer Extruderschnecke verdichtet. Die zähflüssigen Massen werden getrennt einer geeignet gestalteten Strangpreßdüse zugeführt, aus der sie als Schlauch 10 mit innerer Schicht 12 aus Ethylen-Vinylacetat-Copolymer und äußerer Schicht 14 aus Polyurethan austreten.

## Patentansprüche

1. Schlauch für einen medizinischen Behälter,
dadurch gekennzeichnet, daß der Schlauch (10) eine äußere Schicht (12) aus Ethylen-Vinylacetat-Copolymer und eine innere Schicht (14) aus Polyurethan aufweist.

2. Schlauch nach Anspruch 1,
dadurch gekennzeichnet, daß der Vinylacetat-Anteil der äußeren Schicht 10 % bis 30 % beträgt, vorzugsweise 14 % bis 28 %.

3. Schlauch nach Anspruch 1 oder 2,
dadurch gekennzeichnet, daß die äußere Schicht (12) und die innere Schicht (14) fest miteinander verbunden sind.

4. Schlauch nach einem der Ansprüche 1 bis 3,
dadurch gekennzeichnet, daß der Schlauch (10) genau zwei Schichten (12, 14) aufweist.

5. Schlauch nach einem der Ansprüche 1 bis 4,
dadurch gekennzeichnet, daß der Schlauch (10) durch Coextrusion hergestellt ist.

6. Schlauch nach einem der Ansprüche 1 bis 5,
dadurch gekennzeichnet, daß der Schlauch (10) durch Behandlung mit Ethylenoxid oder durch Strahlensterilisation sterilisiert ist.

7. Schlauch nach einem der Ansprüche 1 bis 6,
dadurch gekennzeichnet, daß das Verhältnis der Wandstärke (Wᵢ) der inneren Schicht (12) zur Wandstärke (W) des Schlauches (10) höchstens 0,5 beträgt und vorzugsweise zwischen 0,25 und 0,4 liegt.

8. Schlauch nach einem der Ansprüche 1 bis 7,
dadurch gekennzeichnet, daß die Wandstärke (Wᵢ) der inneren Schicht (12) zwischen 0,2 mm und 0,5 mm beträgt, vorzugsweise 0,35 mm.

9. Schlauch nach einem der Ansprüche 1 bis 8,
dadurch gekennzeichnet, daß die Wandstärke (W) des Schlauches (10) zwischen 1 mm und 3 mm beträgt, vorzugsweise zwischen 1,6 mm und 2 mm.

10. Schlauch nach einem der Ansprüche 1 bis 9,
dadurch gekennzeichnet, daß das Verhältnis des Innendurchmessers (Dᵢ) zum Außendurchmesser (Dₐ) des Schlauches (10) zwischen 0,6 und 0,9 beträgt, vorzugsweise 0,75.

11. Schlauch nach einem der Ansprüche 1 bis 10,
dadurch gekennzeichnet, daß der Außendurchmesser (Dₐ) des Schlauches (10) zwischen 2 mm und 10 mm beträgt, vorzugsweise zwischen 6 mm und 8 mm.

12. Medizinischer Behälter, insbesondere für die enterale oder parenterale Ernährung,
gekennzeichnet durch mindestens einen Ansatz aus einem Schlauch nach einem der Ansprüche 1 bis 11.

13. Medizinischer Behälter nach Anspruch 12,
dadurch gekennzeichnet, daß der Behälter aus Ethylen-Vinylacetat-Copolymer besteht und mit der äußeren Schicht (12) des Schlauches (10) verschweißt ist.

14. Medizinischer Behälter nach Anspruch 12 oder 13,
dadurch gekennzeichnet, daß die innere Schicht (14) des Schlauches (10) mit einem Konnektor oder einem sonstigen Bauteil verklebt ist.

## Claims

1. Hose for a medical container, characterised in that the hose (10) exhibits an outer layer (12) of ethylene vinyl acetate copolymer and an inner layer (14) of polyurethane.

2. Hose according to Claim 1, characterised in that the proportion of vinyl acetate in the outer layer is 10% to 30%, preferably 14% to 28%.

3. Hose according to Claim 1 or 2, characterised in that the outer layer (12) and the inner layer (14) are rigidly connected.

4. Hose according to one of Claims 1 to 3, characterised in that the hose (10) consists of exactly two layers (12, 14).

5. Hose according to one of Claims 1 to 4. characterised in that the hose (10) is manufactured by co-extrusion.

6. Hose according to claims 1 to 5, characterised in that the nose (10) is sterilised by treating it with ethylene oxide or radiation sterilisation.

7. Hose according to one of claims 1 to 6, characterised in that the ratio of the wall thickness (W) of the inner layer (12) to the wall thickness (W) of the hose (10) is a maximum of 0.5, and is preferably between 0.25 and 0.4.

8. Hose according to one of claims 1 to 7, characterised in that the wall thickness (Wᵢ) of the inner layer (12) is between 0.2 mm and 0.5 mm. preferably 0.35 mm.

9. Hose according to one of claims 1 to 8. characterised in that the wall thickness (W) of the nose (10) is between 1 mm and 3 mm, preferably between 1.6 mm and 2 mm.

10. Hose according to one of claims 1 to 9, characterised in that the ratio of the inside diameter (Dᵢ) to the outside diameter (Dₐ) of the hose (10) is between 0.6 and 0.9, preferably 0.75.

11. Hose according to one of claims 1 to 10, characterised in that the outside diameter (Dₐ) of the hose (10) is between 2 mm and 10 mm, preferably between 6 mm and 8 mm.

12. Medical container, particularly for enterat or parenteral nutrition, characterised by at least one shoulder from one hose according to one of claims 1 to 11.

13. Medical container according to Claim 12, characterised in that the container consists of ethylene-vinyl acetate copolymer and is welded to the outer layer (12) of the hose (10).

14. Medical container according to Claim 12 or 13, characterised in that the inner layer (14) of the hose (10) is glued to a connector or other component.

## Revendications

1. Tuyau flexible pour un récipient médical,
caractérisé en ce que le tuyau flexible (10) présente une couche externe (12) constituée d'un copolymère d'éthylène - acétate de vinyle et une couche interne (14) constituée de polyuréthanne.

2. Tuyau flexible selon la revendication 1,
caractérisé en ce que la fraction d'acétate de vinyle de la couche externe s'élève de 10 % à 30 %, de préférence de 14 % à 28 %.

3. Tuyau flexible selon la revendication 1 ou 2,
caractérisé en ce que la couche externe (12) et la couche interne (14) sont reliées à demeure l'une à l'autre.

4. Tuyau flexible selon l'une quelconque des revendications 1 à 3, caractérisé en ce que le tuyau flexible (10) présente exactement deux couches (12, 14).

5. Tuyau flexible selon l'une quelconque des revendications 1 à 4, caractérisé en ce que le tuyau flexible (10) est fabriqué par coextrusion.

6. Tuyau flexible selon l'une quelconque des revendications 1 à 5, caractérisé en ce que le tuyau flexible (10) est stérilisé par traitement avec de l'oxyde d'éthylène ou par stérilisation via exposition à un rayonnement.

7. Tuyau flexible selon l'une quelconque des revendications 1 à 6, caractérisé en ce que le rapport de l'épaisseur de paroi (Wᵢ) de la couche interne (12) à l'épaisseur de paroi (W) du tuyau flexible (10) s'élève au maximum à 0,5 et se situe de préférence entre 0,25 et 0,4.

8. Tuyau flexible selon l'une quelconque des revendications 1 à 7, caractérisé en ce que l'épaisseur de paroi (Wᵢ) de la couche interne (12) se situe entre 0,2 mm et 0,5 mm; de préférence, elle s'élève à 0,35 mm.

9. Tuyau flexible selon l'une quelconque des revendications 1 à 8, caractérisé en ce que l'épaisseur de paroi (W) du tuyau flexible (10) se situe entre 1 mm et 3 mm, de préférence entre 1,6 et 2 mm.

10. Tuyau flexible selon l'une quelconque des revendications 1 à 9, caractérisé en ce que le rapport du diamètre interne (Dᵢ) au diamètre externe (Dₐ) du tuyau flexible (10) se situe entre 0,6 et 0,9; de préférence, il s'élève à 0,75.

11. Tuyau flexible selon l'une quelconque des revendications 1 à 10, caractérisé en ce que le diamètre externe (Dₐ) du tuyau flexible (10) se situe entre 2 mm et 10 mm, de préférence entre 6 mm et 8 mm.

12. Récipient médical, en particulier pour l'alimentation entérale ou parentérale, caractérisé par au moins une tubulure constituée par un tuyau flexible selon l'une quelconque des revendications 1 à 11.

13. Récipient médical selon la revendication 12,
caractérisé en ce que le récipient est constitué d'un copolymère d'éthylène - acétate de vinyle et est soudé avec la couche externe (12) du tuyau flexible (10).

14. Récipient médical selon la revendication 12 ou 13,
caractérisé en ce que la couche interne (14) du tuyau flexible (10) est collée avec un connecteur ou avec un autre élément fabriqué.
